# EUROPÄISCHE PATENTSCHRIFT

(11) **EP 1 468 994 B1**
(45) Veröffentlichungstag und Bekanntmachung des Hinweises auf die Patenterteilung: **24.11.2010**
(21) Anmeldenummer: 04102145.2
(22) Anmeldetag: 24.04.1997
(51) Int. Cl.: C07D 295/10, C07D 295/12, C07D 265/28, C07D 241/04, C07D 211/14

(54) **Verfahren zur Herstellung von Cyclischen Amin substituierten Phenyl-alkyl-Ketonen**
Process for preparing cyclic amine substituted phenyl-alkyl-ketones
Procédé pour la préparation de Phenyl-alkyl-cétones substitués avec une amine cyclique

(30) Priorität: 03.05.1996 CH 113896
(43) Veröffentlichungstag der Anmeldung: 20.10.2004
(62) Teilanmeldung aus: 97810255.6
(73) Patentinhaber: BASF SE, 67056 Ludwigshafen (DE)
(72) Erfinder: Hüsler, Rinaldo, 4055, Basel (CH); Schwabe, Rudolf, 3076, Worb (CH); Luisoli, Reto, 4434, Hölstein (CH)

(56) Entgegenhaltungen:
- EP-A- 0 378 207
- EP-A- 0 401 715
- GB-A- 1 164 608
- US-A- 3 317 538
- CHEMICAL ABSTRACTS, Bd. 108, Nr. 10, 7. März 1988 (1988-03-07), Columbus, Ohio, US; abstract no.: 85724k, BARTULIN ET AL.: "Synthesis and mesogenic properties of ..." XP002038120
- ISHIHARA ET AL.: "Central cholinergic agents ..." CHEM.PHARM.BULL., Bd. 41, Nr. 3, 1993, Seiten 529-538, XP002038119
- BURPITT B E ET AL: "6-(substituted phenyl)-5-methyl-4,5-dihydropyridazin-3(2H )-ones of medicinal interest. The synthesis of SK&F 94836 and SK&F 95654" JOURNAL OF HETEROCYCLIC CHEMISTRY 1988 UNITED STATES, Bd. 25, Nr. 6, 1988, Seiten 1689-1695, XP002286782 ISSN: 0022-152X
- LUNDSTEDT ET AL.: "Synthesis of ..." ACTA CHEM. SCAND., Bd. 38, Nr. 8, 1984, Seiten 717-719, XP009032988
- PATENT ABSTRACTS OF JAPAN Bd. 0031, Nr. 57 (C-068), 22. Dezember 1979 (1979-12-22) -& JP 54 132542 A (MITSUI TOATSU CHEM INC), 15. Oktober 1979 (1979-10-15)

## Beschreibung

Die Erfindung betrifft ein neues Verfahren zur Herstellung von neuen, mit cyclischem Amin substituierte Phenyl-alkyl-Ketonen.

In der EP-B-0284 561 sind α-Aminoacetophenone offenbart, welche als Photoinitiatoren verwendet werden. Hergestellt werden diese Verbindungen durch eine Reihe von Verfahrensschritten, welche im Falle von aromatischen Aminen immer von einem Derivat eines p-Fluorphenylalkylen-1-on ausgehen, wobei in der letzten Synthesestufe das p-ständige Fluor durch eine Aminogruppe ausgetauscht wird. Dieser Austausch erfolgt in einem organischen Lösungsmittel, z.B. Dimethylformamid oder Dimethylsulfoxid, in Gegenwart von Kaliumcarbonat.

Aufgabe der Erfindung war es u.a. einerseits Reaktionen zu entwickeln, welche Fluoraromaten vermeiden, da diese ökologisch problematisch sind, eine unerwünschte Beseitigung der Abfälle verursachen und gegenüber Aminen, wegen ihrer vergleichsweisen hohen Reaktivität, empfindlich sind, und andererseits von organischen Lösungsmitteln wegzukommen, da ein Arbeiten in diesen zu mehr oder weniger dunkel gefärbten Produkten mit Nebenprodukten führt, d.h. einerseits weniger reine Produke und geringere Ausbeuten erhalten werden.

Die aus der Literatur bekannten Verfahren, wonach in einem Alkylphenylketon mit p-ständigem Halogen, vor allem Fluor oder Chlor, im Phenylkern das Halogen gegen einen Aminrest ausgetauscht wird, arbeiten:
a) in einem organischen Lösungsmittel (z.B. EP-B-0138754, Umsetzung von 1-(4-Fluorphenyl)-2-methyl-propanon-1 mit Piperidin in Dimethylsulfoxid; CH 200 365, Umsetzung von p-Chlorstearophenon mit Dimethylamin in Ethanol in Gegenwart von Kupferpulver als Katalysator; T. Ibata, Y. Isogami, J. Toyoda, Bull. Chem. Soc. Jpn. 64(1)42-49 (1991), Umsetzung von Chloracetophenon mit Pyrrolidin in Tetrahydrofuran unter Anwendung extrem hoher Drucke (7,2 Kbar); J. Org. Chem. 31(7), 2319-21 (1966), Umsetzung von 1-(4-Fluorphenyl)-propan-1-on mit alicyclischen Aminen, wie Piperidin, in Dimethylformamid oder Dimethylsulfoxid, oder
b) ohne Lösungsmittel (z.B. G. Kresze und H. Goetz, Chem. Berichte 90, 2161, 2174 (1957)), Umsetzung von p-Bromacetophenon mit Piperidin unter Rückfluss mit Ausbeuten an 1-(4-piperidinophenyl)-ethanon von 19 %; oder
c) in Wasser (z.B.: T. Lundstedt, P. Thoren, R. Carlson, Acta Chemica Scand. B 38, 1984 No. 8 S. 717-719; Umsetzung von p-Chloracetophenon mit Dimethylamin unter Druck in Wasser; US-A-1'946'058, Umsetzung von p-Chloracetophenon mit wässrigem Ammoniak in Wasser unter Druck in Gegenwart von Kupferoxid als Katalysator; JP 78-40404, Umsetzung von p-Chloracetophenon mit Mono- oder Dialkylaminen in Wasser unter Druck in Gegenwart von Kupferpulver als Katalysator) wo einerseits Explosionen auftraten und andererseits die Ausbeuten unter 80 % liegen.

Es wurde nun gefunden, dass überraschenderweise unter bestimmten Voraussetzungen eine Reaktion von p-Halogenphenylalkylketonen, vor allem den entsprechenden p-Brom- und p-Chlorverbindungen, mit Aminen, vor allem cyclischen Aminen, in Wasser sehr selektiv und gut mit hohen Ausbeuten verläuft.

Derartige Phenyl-alkyl-ketone, welche in p-Stellung im Phenylkern durch ein cyclisches Amin substituiert sind, und zudem in α-Stellung zur Ketogruppe eine freie Methylengruppe besitzen sind nur wenig bekannt; verwiesen wird z.B. auf die EP-B-0'138'754 (2-Methyl-1(4-piperidinophenyl)-propanon-1); CH 200365 (p-Dimethylaminostearophon, wobei anstelle des Dimethylaminorestes gemäss Beschreibung auch Piperidin möglich ist, ohne aber ein konkretes Beispiel zu geben); G. Kresze und H. Goetz, Chem. Berichte 90, 2161, 2174 (1957), (1-(4-Piperidinophenyl)-ethanon); T. Ibata, Y. Isogami, J. Toyoda, Bull. Chem. Soc. JPn. 64(1), 42-49 (1991), (1-(4-Pyrrolidon)acetophenon); und J. Org. Chem. 31(7), 2319-21 (1966), (1-(4-Piperidinophenyl)-propanon-1).

Die Erfindung, und gleichzeitig die Lösung der gestellten Aufgabe, betrifft nun sowohl neue mit cyclischem Amin p-substituierte Phenyl-alkyl-ketone, welche u.a. als neue Zwischenprodukte zur Herstellung von bestimmten Photoinitiatoren verwendet werden können, als auch ein neues Verfahren zur Herstellung dieser Zwischenprodukte.

Bei den neuen, erfindungsgemässen, mit cyclischem Amin p-substituierten Phenyl-alkyl-Ketonen handelt es sich um Verbindungen der Formel (I) 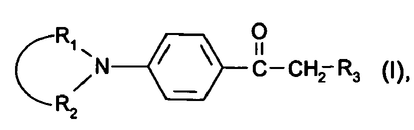 worin bedeuten:
R₁ und R₂ zusammen unverzweigtes oder verzweigtes, unsubstituiertes oder substituiertes C₃-C₂₀-Alkylen, welches durch ein oder mehrere -O-, -S- oder -N(R₄)-Gruppen unterbrochen sein kann,
R₃ unverzweigtes oder verzweigtes, unsubstituiertes oder substituiertes C₂-C₂₀-Alkyl, und R₄ Wasserstoff, unverzweigtes oder verzweigtes C₁-C₃-Alkyl, unverzweigtes oder verzweigtes C₃-C₅-Alkenyl, C₇-C₉-Phenylalkyl, C₁-C₄-Hydroxyalkyl oder Phenyl, wobei wenn R₁ und R₂ zusammen unsubstituiertes Tetramethylen bedeuten, R₃ nicht unsubstituiertes C₆-Alkyl bedeutet.

Bedeuten R₁ und R₂ zusammen einen C₃-C₂₀-Alkylenrest so handelt es sich, durch den Einschluss des N-Atoms, um ein heterocyclisches Ringsystem. Dieses N-heterocyclische Ringsystem kann zudem noch unterbrochen sein durch ein oder mehrere weitere Heteroatome wie -O-, -S- und/oder -N(R₄)-Gruppen und es kann zudem noch ein- oder mehrmals substituiert sein.

Als C₃-C₂₀-Alkylenreste kommen sowohl unverzweigte als auch verzweigte Alkylenreste in Betracht und als Substituenten z.B. Hydroxy, C₁-C₄-Alkoxy, Hydroxymethyl, C₁-C₄-Alkoxymethyl, -COO(C₁-C₄-Alkyl) oder auch Phenyl.

Unverzweigte oder verzweigte C₃-C₂₀-Alkylenreste sind beispielsweise Tri-, Tetra-, Penta-, Hexa-, Hepta-, Octa-, Deca-, Dodeca- oder Octadecamethylen, und 2,2-Dimethyltrimethylen oder 1,3,3-Trimethyltetramethylen.

Durch Sauerstoff, Schwefel oder -N(R₄)- unterbrochenes C₃-C₂₀-Alkylen kann ein- oder mehrfach unterbrochen sein und bedeutet beispielsweise:
-CH₂-CH₂-O-CH₂-CH₂-, -CH₂-CH(CH₃)-O-CH(CH₃)-CH₂-,
-CH₂-CH₂-(O-CH₂-CH₂-)₂-O-CH₂-CH₂-, -CH₂-CH₂-(O-CH₂-CH₂-)₃-O-CH₂-CH₂-,
-CH₂-CH₂-(O-CH₂-CH₂-)₄-O-CH₂-CH₂-, -CH₂-CH₂-O-CH₂-CH₂-NH-CH₂-CH₂-O-CH₂-CH₂-,
-CH₂-CH₂-(O-CH₂-CH₂-)₂-NH-CH₂-CH₂-O-CH₂-CH₂-,
-CH₂-CH₂-(O-CH₂-CH₂-)₂-NH-(CH₂-CH₂-O-)₂-CH₂-CH₂-, -CH₂-CH₂-S-CH₂-CH₂-,
-CH₂-CH₂-NH-CH₂-CH₂-, -CH₂-CH₂-N(CH₃)-CH₂-CH₂-, -CH(CH₃)-CH₂-NH-CH(CH₃)-CH₂-,
-CH₂-CH₂-NH-CH₂-CH₂-NH-CH₂-CH₂-, -CH₂-CH₂-CH₂-NH-CH₂-CH₂-CH₂-NH-CH₂-CH₂-CH₂-,
-CH₂-CH₂-(NH-CH₂-CH₂-)₂-NH-CH₂-CH₂-, -CH₂-CH₂-(NH-CH₂-CH₂-)₄-NH-CH₂-CH₂-,
-CH₂-CH₂-NH-CH₂-CH₂-CH₂-NH-CH₂-CH₂-NH-CH₂-CH₂-CH₂- oder
-CH₂-CH₂-CH₂-NH-CH₂-CH₂-CH₂-NH-CH₂-CH₂-NH-CH₂-CH₂-CH₂-.

Es handelt sich bei R₁ und R₂ unter Einschluss des N-Atoms beispielsweise um folgende heterocyclischen Reste:

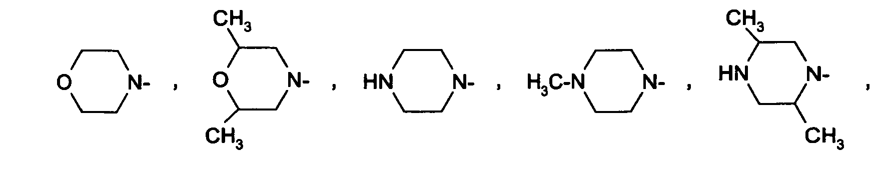

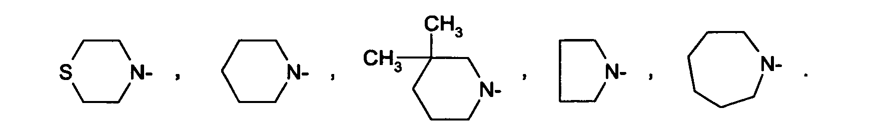

Sofern es sich beim heterocyclischen Rest um ein 6-Ringsystem handelt, darf dieses in 6-Stellung 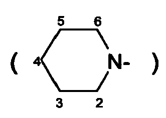 nicht substituiert sein.

Bevorzugt ist ein 6-Ringsystem, vor allem Morpholin.

Bedeutet R₃ einen unsubstituierten oder substituierten C₂-C₂₀-Alkylrest, so kann auch dieser unverzweigt oder verzweigt sein. Beispielsweise handelt es sich um folgende Alkylreste: Ethyl, Propyl, n-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Isopropyl, sec-Butyl, Isobutyl, tert-Butyl, 2-Ethylbutyl, Isopentyl, 1-Methylpentyl, 1,3-Dimethylbutyl, 1-Methylhexyl, Isoheptyl, 1,1,3,3-Tetramethylbutyl, 2,2,4,4-Tetramethylbutyl, 1-Methylheptyl, 3-Methylheptyl, 2-Ethylhexyl, 1,1,3-Trimethylhexyl, 1,1,3,3-Tetramethylpentyl, Isodecyl, 1-Methylundecyl oder 1,1,3,3,5,5-Hexamethylhexyl.

Diese C₂-C₂₀-Alkylreste können ferner noch ein- oder mehrmals substituiert sein, z.B. durch Cyclohexyl, Phenyl, C₁-C₄-Alkoxy oder Phenoxy.

Es handelt sich dann beispielsweise um die R₃-Reste: 2-Methoxyethyl, 3-Butoxypropyl, 2-Isopropoxyethyl, 4-Phenoxybutyl, 2-Phenylethyl oder 3-Phenylpropyl.

Besonders bevorzugte R₃ Alkylreste sind unsubstituierte, unverzweigte oder verzweigte Alkylreste mit 2 bis 10 Kohlenstoffatomen, vor allem solche mit 2 bis 7 Kohlenstoffatomen, insbesondere mit 2 bis 5 Kohlenstoffatomen wie Ethyl oder Propyl.

Bedeutet R₄ C₁-C₃-Alkyl so kann dieses unverzweigt oder verzweigt sein; z.B. handelt es sich um Methyl, Ethyl, n- und iso-Propyl.

Bedeutet R₄ C₃-C₅-Alkenyl so handelt es sich um unverzweigtes oder verzweigtes Alkenyl wie beispielsweise um Propenyl, Allyl, Butenyl wie 2-Butenyl, 3-Butenyl und Isobutenyl, und um Pentenyl wie n-2,4-Pentadienyl.

Bedeutet R₄ C₇-C₉-Phenylalkyl so handelt es sich z.B. um Benzyl, α-Methylbenzyl, α,α-Dimethylbenzyl oder 2-Phenylethyl.

Bedeutet R₄ C₁-C₄-Hydroxyalkyl, so handelt es sich z.B. um 2-Hydroxyethyl, 2-Hydroxypropyl oder um 2-Hydroxyisobutyl.

In den bevorzugten Verbindungen der Formel I bedeutet R₄ Wasserstoff, C₁-C₃-Alkyl, Allyl, Benzyl oder C₂-C₃-Hydroxyalkyl und vor allem Wasserstoff oder Methyl.

Bevorzugte Verbindungen entsprechen der Formel I worin bedeuten:
R₁ und R₂ zusammen unsubstituiertes oder durch Hydroxy, C₁-C₄-Alkoxy, Hydroxymethyl, C₁-C₄-Alkoxymethyl, -COO(C₁-C₄-Alkyl) oder Phenyl substituiertes C₃-C₂₀-Alkylen, welches durch ein oder mehrere -O-, -S- oder -N(R₄)-Gruppen unterbrochen sein kann,
R₃ unsubstituiertes oder durch C₁-C₄-Alkoxy, Phenoxy, Cyclohexyl oder Phenyl substituiertes C₂-C₂₀-Alkyl, und
R₄ Wasserstoff, C₁-C₃-Alkyl, C₃-C₅-Alkenyl, C₇-C₉-Phenylalkyl, C₁-C₄-Hydroxyalkyl oder Phenyl,
vor allem solche Verbindungen worin bedeuten:
R₁ und R₂ zusammen unverzweigtes oder verzweigtes C₄-C₁₂-Alkylen, das durch eine -O-, -S- oder -N(R₄)-Gruppe unterbrochen sein kann,
R₃ C₂-C₁₀-Alkyl, und
R₄ Wasserstoff, C₁-C₃-Alkyl, Allyl, Benzyl oder C₂-C₃-Hydroxyalkyl,
oder worin bedeuten:
R₁ und R₂ zusammen unverzweigtes oder verzweigtes C₄-C₈-Alkylen unter Bildung eines 6-gliedrigen Ringes, der durch eine -O-, -S- oder -N(R₄)-Gruppe unterbrochen sein kann,
R₃ C₂-C₇-Alkyl, und
R₄ Wasserstoff oder Methyl,
und insbesondere solche worin bedeuten:
R₁ und R₂ zusammen unter Einschluss des N-Atoms einen 6-gliedrigen Ring, welcher noch durch eine -O-, -S- oder -N(R₄)-Gruppe unterbrochen sein kann,
oder worin bedeuten:
R₁ und R₂ zusammen unter Einschluss des N-Atoms einen Morpholinyl-, Dimethylmorpholinyl-, Piperazinyl-, N-Methylpiperazinyl- oder 2,5-Dimethylpiperazinylrest, und insbesondere solche worin R₁ und R₂ zusammen unter Einschluss des N-Atoms den Morpholinylrest bilden.

Die Herstellung der Verbindungen der Formel I erfolgt nach einem neuen Verfahren, welches einen weiteren Gegenstand der Erfindung darstellt.

Dieses erfindungsgemässe Verfahren zur Herstellung von Verbindungen der Formel I 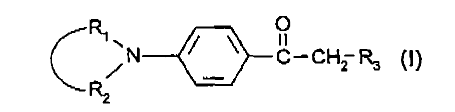 worin bedeuten:
R₁ und R₂ zusammen unverzweigtes oder verzweigtes, unsubstituiertes oder substituiertes C₃-C₂₀-Alkylen, welches durch ein oder mehrere -O-, -S- oder -N(R₄)-Gruppen unterbrochen sein kann,
R₃ unverzweigtes oder verzweigtes, unsubstituiertes C₂-C₁₀-Alkyl, und
R₄ Wasserstoff, unverzweigtes oder verzweigtes C₁-C₃-Alkyl, unverzweigtes oder verzweigtes C₃-C₅-Alkenyl, C₇-C₉-Phenylalkyl, C₁-C₄-Hydroxyalkyl oder Phenyl, besteht in der Aminolyse eines p-Halogenphenylalkylketons der Formel II 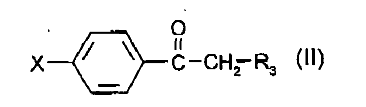 mit einem cyclischen Amin der Formel III

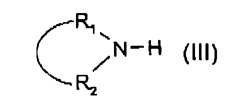

in Wasser bei einer Temperatur von mindestens 130°C und, dadurch gekennzeichnet, dass die Reaktion unter Druck in einem Druckkessel durchgeführt wird,
in welchen Formeln X ein Halogenatom bedeutet und R₁, R₂ und R₃ die unter Formel I angegebene Bedeutung haben.

Vorzugsweise wird als p-Halogenphenylalkylketon der Formel II ein solches verwendet worin X Brom und insbesondere Chlor bedeutet.

Vorteilhaft ist die Menge an cyclischem Amin der Formel III im Ueberschuss, bezogen auf das p-Halogenphenylalkylketon der Formel II, vorhanden. Dieser Ueberschuss beträgt vor allem etwa 2,5-20 insbesondere 2,5-12 Moläquivalente.

Die Menge an Wasser liegt zwischen etwa 1 bis 100 vorzugsweise 2 bis 20 und insbesondere 2,5 bis 10 Moläquivalenten bezogen auf 1 Moläquivalent p-Halogenphenylalkylketon der Formel II; grössere Wassermengen sind aber auch nicht kritisch.

Die Umsetzung wird vorteilhafterweise unter Druck (ca. 3-30 bar) in einem Druckkessel, vor allem in einem Hochdruckreaktor aus Stahl mit Flügelrührer, Manometer und Thermoelement durchgeführt. Es ist aber auch möglich die Reaktion ohne Druckkessel unter Rückfluss (ca. 105°C - 110°C) durchzuführen.

Zweckmässig liegt die Temperatur in einem Temperaturbereich von etwa 140°C bis 240°C insbesondere 150°C bis 230°C. Arbeitet man mit dem p-Bromphenylalkylketon der Formel II so liegt der Temperaturbereich zwischen etwa 140°C und 200°C vorteilhaft zwischen 160°C - 180°C, und arbeitet man mit dem p-Chlorphenylalkylketon der Formel II so liegt der Temperaturbereich zwischen etwa 180°C und 240°C, vorteilhaft zwischen 200°C und 230°C.

Katalysatoren können, müssen aber nicht zugesetzt werden; diese beschleunigen die Reaktion zwar in einem gewissen Ausmass, aber andererseits vermindert ein Arbeiten ohne Katalysator ökologische Probleme und relativisiert die Vorteile eines Schwermetallzusatzes.

Als Katalysatoren kommen vor allem in Betracht:
Kupfer- oder Nickelverbindungen oder deren Salze, wie Kupfer(I)chlorid, Kupfer(I)bromid, Kupfer(I)jodid, Kupfer(II)bromid, Kupfer(II)chlorid, Kupfercarbonat, Kupfer(II)sulfat, Kupferoxid sowie Kupferpulver, oder Nickelacetat, Nickeloxid, Nickelchlorid und Nickelbromid.

Diese Katalysatoren werden in Mengen von etwa 0,1-15 Gew.%, insbesondere 0,5 - 5 Gew.% bezogen auf 100,0 Gew.% p-Halogenphenylalkylketon der Formel II zugesetzt.

Weitere Lösungsmittel sind im Prinzip zur Durchführung der Umsetzung nicht nötig, können aber mitverwendet werden; zweckmässig haben sich dabei hochsiedende und polare Lösungsmittel bewährt wie Diethylenglykol, Diethylenglykolmonomethylether, Diethylenglykoldimethylether, Benzylalkohol, Phenylethylalkohol oder Phenoxyethanol.

Die Umsetzung des cyclischen Amins der Formel III mit dem p-Halogenphenylalkylketon der Formel 11 wird vorteilhafterweise derart durchgeführt, dass entweder:
a) das p-Halogenphenylalkylketon der Formel II im Reaktionsgefäss zusammen mit dem Wasser und dem cyclischen Amin vorgelegt wird und sofort bis zur Endtemperatur aufgeheizt wird, oder
b) das p-Halogenphenylalkylketon der Formel II wird im Reaktionsgefäss zusammen mit dem Wasser und dem Amin vorgelegt und langsam über Stunden während der Reaktion bis zur Endtemperatur aufgeheizt, oder
c) das p-Halogenphenylalkylketon der Formel II wird während der Reaktion, zweckmässig geschmolzen, zum Wasser und dem cyclischen Amin, das vorgängig auf die Reaktionstemperatur aufgeheizt wurde, zudosiert. Diese Verfahrensvariante reduziert oder eliminiert vor allem das Risiko einer autokatalytischen Zersetzung bei sehr hohen Temperaturen. Beispielsweise kann so verfahren werden, dass alle Komponenten im Reaktionsgefäss vorgelegt werden und das p-Bromphenylalkylketon der Formel II in einem Temperaturbereich von etwa 140°C-190°C während Stunden zudosiert wird, wobei die Temperatur während etwa 3-12 Stunden vom tieferen Niveau allmählich aufs höhere Niveau erhöht wird, oder, dass das p-Chlorphenylalkylketon der Formel II in einem Temperaturbereich von etwa 180°C-230°C während Stunden zudosiert wird, wobei die Temperatur während etwa 3-12 Stunden vom tieferen Niveau allmählich aufs höhere Niveau erhöht wird.

Aus Sicherheitsgründen ist es zweckmässig die Akkumulation des p-Halogenphenylalkylketons unter Kontrolle zu halten.

Bevorzugte Verfahrens-Arbeitsweisen bestehen z.B. darin, dass 1 Teil (Teile sind hier und im folgenden auf Molmengen bezogen) p-Bromphenylalkylketon bzw. 1 Teil p-Chlorphenylalkylketon der Formel II worin R₃ unverzweigtes oder verzweigtes, unsubstituiertes C₂-C₇-Alkyl bedeutet mit 5 Teilen eines cyclischen Amins der Formel III worin R₁ und R₂ zusammen C₄-C₆-Alkylen bedeuten, das durch eine -O-, -S- oder -N(R₄)-Gruppe unterbrochen sein kann und R₄ Wasserstoff oder Methyl bedeutet und 5 Teile Wasser im Reaktionsgefäss vorgelegt werden und unter Druck bei einer Temperatur von etwa 160°C - 180°C bzw. 200°C - 230°C umgesetzt werden oder, dass 10 bis 20 Teile eines cyclischen Amins der Formel III, worin R₁ und R₂ zusammen C₄-C₆-Alkylen bedeuten, das durch eine -O-, -S- oder -N(R₄)-Gruppe unterbrochen sein kann und R₄ Wasserstoff oder Methyl bedeutet und 20 bis 40 Teile Wasser im Reaktionsgefäss vorgelegt werden, 2 bis 4 Teile p-Chlorphenylalkylketon der Formel II, worin R₃ unverzweigtes oder verzweigtes, unsubstituiertes C₂-C₇-Alkyl bedeutet zudosiert werden und unter Druck bei etwa 210°C - 230°C umgesetzt werden.

Die Aufarbeitung und Reinigung der neuen Phenyl-alkyl-Ketone der Formel I, welche mit einem cyclischen Amin substituiert sind, erfolgt nach bekannten Methoden, z.B. mittels Destillation, Kristallisation und Filtration.

Die cyclischen Amine der Formel III sind bekannt, teilweise im Handel erhältlich, und können nach bekannter Art und Weise hergestellt werden (z.B. Houben-Weyl, Vol. 11/1 (1957) S. 26-29, 32-33 und 63-67; Org. Synth. Coll. Vol. 3, 307 (1955); JACS 109, 1496-1502 (1987) oder Tetrahedron Vol. 40, 1433-1456 (1984).

Beispielsweise handelt es sich um folgende Verbindungen: Morpholin, Piperidin, Pyrrolidin, Piperazin, N-Methylpiperazin, 2,6-Dimethylmorpholin, Dimethylpiperidin, Dimethylpiperazin, Thiomorpholin, 4-Hydroxypiperidin, 3-Ethoxycarbonylpiperidin oder Hexamethylenimin.

Die p-Halogenphenylalkylketone der Formel II sind ebenfalls bekannt (z.B. Friedel-Crafts and related Reactions, Ed. C.A. Olah, J. Wiley and Sons, N.Y. (1964) Vol. 3, Parts 1+2; Chem. Rev. 55, 229 (1955); Org. Synth. Coll. Vol. 3, 14 (1955) und JACS 109,7122 (1987).

Beispiele für einzelne Verbindungen sind: 1-(4-Bromphenyl)-n-butan-1-on, -n-pentan-1-on, - n-hexan-1-on, -n-heptan-1-on, -n-octan-1-on, -iso-nonan-1-on, 1-(4-Chlorphenyl)-n-butan-1-on und 1-(4-Chlorphenyl)-n-pentan-1-on.

Die Herstellung der p-Halogenphenylalkylketone der Formel II erfolgt nach bekannter Art und Weise z.B. mittels einer Friedel-Crafts-Reaktion aus einem Halogenbenzol und einem Alkancarbonsäurechlorid nach folgendem Reaktionsschema:

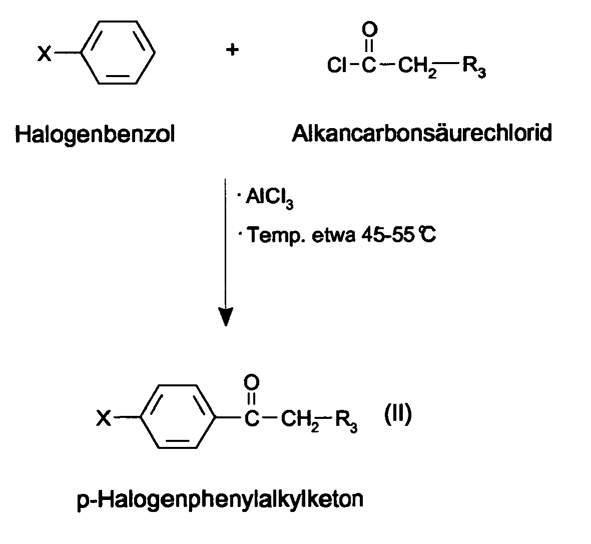

In diesen Formeln bedeutet X ein Halogenatom, vorzugsweise Chlor oder Brom, und R₃ hat die weiter vorne angegebene Bedeutung.

Beide Edukte, das Halogenbenzol und das Alkancarbonsäurechlorid, sind bekannt.

Beispiele für Halogenbenzole sind vor allem das Monobrombenzol und insbesondere das Monochlorbenzol.

Beispiele für Alkancarbonsäurechloride sind z.B. Buttersäurechlorid, Isobuttersäurechlorid, n-Valeriansäurechlorid, Isovaleriansäurechlorid, Capronsäurechlorid, Oenanthsäurechlorid, Caprylsäurechlorid, Pelargonsäurechlorid, Caprinsäurechlorid, Laurinsäurechlorid, Myristinsäurechlorid, Palmitinsäurechlorid, Stearinsäurechlorid, Arachinsäurechlorid, Eikosancarbonsäurechlorid und Behensäurechlorid.

Das gemäss der Umsetzung erhaltene p-Halogenphenylalkylketon der Formel II muss vor dessen weiteren Umsetzung mit dem cyclischen Amin der Formel III isoliert werden.

Es ist überraschend, und war aus der eingangs genannten Literatur nicht vorhersehbar, dass der Zusatz von Wasser zur Reaktion des p-Halogenphenylalkylketons der Formel II mit dem cyclischen Amin der Formel III sehr effizient die Bildung von farbigen Nebenprodukten und Verharzungen verhindert, und sehr reine, helle Produkte, die eine Reinheit von >99,0 % besitzen, erhalten werden.

Zudem bringt ein Arbeiten in Wasser, vor allem grosstechnisch, im Vergleich zu organischen Lösungsmitteln, wie Dimethylsulfoxid oder Dimethylformamid, ökologische Vorteile mit sich.

Es ist weiterhin überraschend, dass die Reaktion des p-Halogenphenylalkylketons der Formel II mit dem cyclischen Amin der Formel III, d.h. der Halogen-Austausch bei einem wenig aktivierten Benzolderivat so glatt und so schnell in Wasser verläuft.

Ferner ist es auch überraschend, dass die Aminolyse-Reaktion ohne zwingenden Zusatz eines Katalysators verläuft und hohe Ausbeuten von 88 % bis 96 % erzielt werden; durch die Abwesenheit eines Katalysators erspart man sich auch die Entfernung desselben aus dem Endprodukt, welches meistens ein zeitaufwendiges Verfahren ist.

Verwendet werden die Verbindungen der Formel I 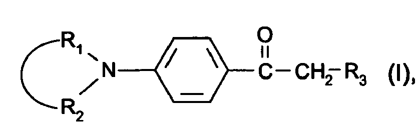 worin bedeuten:
R₁ und R₂ zusammen unverzweigtes oder verzweigtes, unsubstituiertes oder substituiertes C₃-C₂₀-Alkylen, welches durch ein oder mehrere -O-, -S- oder -N(R₄)-Gruppen unterbrochen sein kann,
R₃ unverzweigtes oder verzweigtes, unsubstituiertes oder substituiertes C₂-C₂₀-Alkyl, und
R₄ Wasserstoff, unverzweigtes oder verzweigtes C₁-C₃-Alkyl, unverzweigtes oder verzweigtes C₃-C₅-Alkenyl, C₇-C₉-Phenylalkyl, C₁-C₄-Hydroxyalkyl oder Phenyl, bzw. die Verbindungen erhalten gemäss dem genannten erfindungsgemässen Verfahren, vor allem zur Herstellung von radikalischen Photoinitiatoren der Formel IV 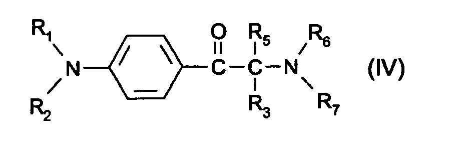
oder deren Säureadditionssalzen, worin bedeuten:
R₁ und R₂ zusammen unverzweigtes oder verzweigtes, unsubstituiertes C₃-C₂₀-Alkylen, das durch ein oder mehrere -O-, -S- oder -N(R₄)-Gruppen unterbrochen, und/oder mit Hydroxy, C₁-C₄-Alkoxy, Hydroxymethyl, C₁-C₄-Alkoxymethyl, -COO(C₁-C₄-Alkyl) oder Phenyl substituiert sein kann;
R₃ unverzweigtes oder verzweigtes, unsubstituiertes oder durch C₁-C₄-Alkoxy, Phenoxy, Cyclohexyl oder Phenyl substituiertes C₂-C₂₀-Alkyl,
R₄ Wasserstoff, C₁-C₃-Alkyl, C₃-C₅-Alkenyl, C₇-C₈-Phenylalkyl, C₁-C₄-Hydroxyalkyl oder Phenyl;
R₅ entweder
   (a) ein Rest der Formel 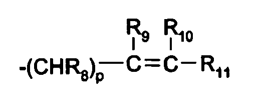 worin p null oder 1 ist,
      oder
   (b) ein Rest der Formel 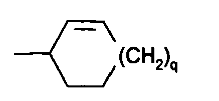 ist, wobei q 0, 1, 2 oder 3 bedeutet
      oder
   c) ein Rest der Formel 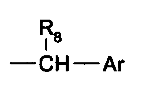
   worin Ar einen unsubstituierten oder durch Halogen, OH, C₁-C₁₂-Alkyl oder durch OH, Halogen, -N(R₁₂)₂, -C₁-C₁₂-Alkoxy, -COO(C₁-C₁₈-Alkyl), -CO(OCH₂CH₂)ₙOCH₃ oder -OCO(C₁-C₄)-Alkyl substituiertes C₁-C₄-Alkyl, oder durch -COO(C₁-C₁₈-Alkyl) oder -CO(OCH₂CH₂)ₙOCH₃ substituiertes C₁-C₄-Alkoxy; -(OCH₂CH₂)ₙOH, -(OCH₂CH₂)ₙOCH₃, C₁-C₈-Alkylthio, Phenoxy, -COO(C₁-C₁₈-Alkyl), -CO(OCH₂CH₂)ₙOCH₃, Phenyl oder Benzoyl substituierten Phenyl-, Naphthyl-, Furyl-, Thienyl- oder Pyridylrest bedeutet, worin n 1 - 20 ist,
in welchen Formeln
R₁₂ Wasserstoff, C₁-C₈-Alkyl, C₃-C₅-Alkenyl, C₇-C₉-Phenylalkyl, C₁-C₄-Hydroxyalkyl oder Phenyl bedeutet,
R₈ Wasserstoff, C₁-C₈-Alkyl oder Phenyl bedeutet, und
R₉, R₁₀ und R₁₁ unabhängig voneinander Wasserstoff oder C₁-C₄-Alkyl bedeuten oder R₉ und R₁₀ zusammen C₃-C₇-Alkylen sind,
R₆ Wasserstoff, C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl oder C₇-C₉-Phenylalkyl bedeutet, R₇ C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl, C₇-C₉-Phenylalkyl, Phenyl oder durch Halogen, C₁-C₁₂-Alkyl, C₁-C₄-Alkoxy oder -COO(C₁-C₄-Alkyl) substituiertes Phenyl bedeutet oder R₇ zusammen mit R₃ C₁-C₇-Alkylen, C₇-C₁₀-Phenylalkylen, o-Xylylen, 2-Butenylen oder C₂-C₃-Oxa- oder Azaalkylen bedeutet, oder
R₆ und R₇ zusammen C₃-C₇-Alkylen bedeuten, das durch -O-, -S-, -CO- oder -N(R₁₃)-unterbrochen sein kann oder durch Hydroxy, C₁-C₄-Alkoxy oder -COO(C₁-C₄-Alkyl) substituiert sein kann, wobei R₁₃ Wasserstoff, C₁-C₁₂-Alkyl, das durch ein oder mehrere -O-unterbrochen sein kann, C₃-C₅-Alkenyl, C₇-C₉-Phenylalkyl, C₁-C₄-Hydroxyalkyl, -CH₂CH₂CN, -CH₂CH₂COO(C₁-C₄-Alkyl), C₂-C₈-Alkanoyl oder Benzoyl bedeutet.

Das erfindungsgemässe Verfahren erlaubt so auf einfache, grosstechnisch sehr gut realisierbare, Art und Weise die Herstellung von Photoinitiatoren der Formel IV ausgehend von Monohalogenbenzol und einem Säurechlorid der Formel 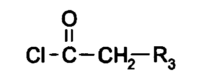 mittels einer Friedel-Crafts-Reaktion zu einem p-Halogenphenylalkyl-Keton der Formel II und dessen Aminolyse,
mit einem cyclischen Amin der Formel III 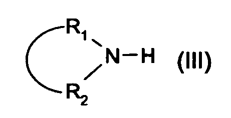 in Wasser bei einer Temperatur von mindestens 130°C in welchen Formeln X ein Halogenatom bedeutet und R₁, R₂ und R₃ die oben angegebene Bedeutung haben, zu einem cyclischen Amin substituierten Phenyl-alkyl-Keton der Formel I 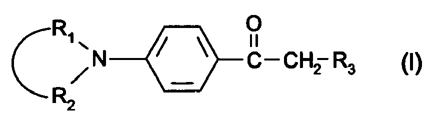 Halogenierung dieser Phenyl-alkyl-Keton-Verbindung der Formel I, Umsetzung mit einem Amin der Formel 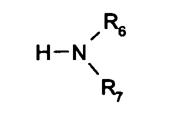 anschliessender Umsetzung mit einer den Rest R₅ einführenden Verbindung und Stevens-Umlagerung unter basischen Konditionen.

Bei der Halogenierung der Phenyl-alkyl-Keton Verbindung der Formel I handelt es sich um eine α-Halogenierung mit z.B. Brom oder Chlor in einem Lösungsmittel wie z.B. Eisessig bei Raumtemperatur. Die anschliessende Aminierung mit einem Amin der Formel 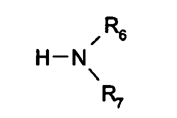 worin R₆ und R₇ die weiter oben angegebene Bedeutung haben (z.B. Dimethylamin) wird in einem geeigneten Lösungsmittel z.B. Methylethylketon durchgeführt. Anschliessend an die Aminierung erfolgt die Umsetzung mit einer die Gruppe R₅ einführenden Verbindung beispielsweise Benzylbromid, Benzylchlorid, Allylbromid oder Allylchlorid und daran anschliessend die Stevens-Umlagerung unter basischen Bedingungen, z.B. NaOH oder KOH.

Durch das Vorhandensein einer basischen Aminogruppe lassen sich die Photoinitiatoren der Formel IV durch Addition von Säuren in die entsprechenden Säureadditionssalze überführen. Die Säuren können anorganische oder organische Säuren sein. Beispiele für solche Säuren sind HCl, HBr, H₂SO₄, H₃PO₄, Mono- oder Polycarbonsäuren wie z.B. Essigsäure, Oelsäure, Bernsteinsäure, Sebacinsäure, Weinsäure oder CF₃COOH, Sulfonsäuren wie z.B. CH₃SO₃H, C₁₂H₂₅SO₃H, p-C₁₂H₂₅-C₆H₄-SO₃H, p-CH₃-C₆H₄-SO₃H oder CF₃SO₃H, Acrylsäure, Methacrylsäure, Polyacrylsäure, Polymethacrylsäure und Benzoesäure.

Photoinitiatoren für radikalisch polymerisierbare Verbindungen sind Verbindungen, welche bei Bestrahlung mit kurzwelligem Licht in radikalische Bruchstücke zerfallen, welche die eigentlichen Initiatoren für die Polymerisation der ethylenisch ungesättigten Verbindungen sind.

Diese Photoinitiatoren werden vor allem verwendet für die Photopolymerisation ethylenisch ungesättigter Verbindungen bzw. Gemischen, die solche Verbindungen enthalten, für die Photohärtung von pigmentierten Systemen wie Druckfarben oder Weisslacke, für die Photohärtung von unpigmentierten Systemen, wie UV-härtbare Druckfarben, für die Herstellung von Photoresists und Druckplatten und für Aussenanstriche, die im Tageslicht oberflächlich nachhärten.

Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkyl-acrylate oder -methacrylate, wie z.B. Methyl- , Ethyl-, Butyl-, 2-Ethylhexyl- oder 2-Hydroxyethylacrylat, Isobornylacrylat, Methyl- oder Ethylmethacrylat. Weitere Beispiele hierfür sind Acrylnitril, Acrylamid, Methacrylamid, N-substituierte (Meth)acrylamide, Vinylester wie Vinylacetate, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol-, oder Bisphenol-A-diacrylat, 4,4'-Bis(2-acryloyloxyethoxy)-diphenylpropan, Trimethylolpropan-triacrylat, Pentaerythrittriacrylat oder -tetraacrylat, Vinylacrylat, Divinylbenzol, Divinylsuccinat, Diallylphthalat, Triallylphosphat, Triallylisocyanurat oder Tris-(2-acryloyloxyethyl)isocyanurat.

Beispiele für höhermolekulare (oligomere) mehrfach ungesättigte Verbindungen sind acrylierte Epoxidharze, acrylierte Polyether, acrylierte Polyurethane oder acrylierte Polyester. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Solche ungesättigten Oligomere kann man auch als Prepolymere bezeichnen.

Häufig verwendet man Zweikomponenten-Gemische eines Prepolymeren mit einem mehrfach ungesättigten Monomeren oder Dreikomponentengemische, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass man ein Lösungsmittel verwenden muss.

Solche Zwei- und Dreikomponentensysteme auf der Basis eines Prepolymeren werden sowohl für Druckfarben als auch für Lacke, Photoresists oder andere photohärtbare Massen verwendet. Als Bindemittel für Druckfarben werden vielfach auch EinkomponentenSysteme auf der Basis photohärtbarer Prepolymerer verwendet.

Ungesättigte Polyesterharze werden meist in Zweikomponentensystemen zusammen mit einem einfach ungesättigten Monomer, vorzugsweise mit Styrol, verwendet. Für Photoresist werden oft spezifische Einkomponentensysteme verwendet, wie z.B. Polymaleinimide, Polychalkone oder Polyimide, wie sie in der DE-OS 2 308 830 beschrieben sind.

Die ungesättigten Verbindungen können auch im Gemisch mit nicht-photopolymerisierbaren filmbildenden Komponenten verwendet werden. Diese können z.B. physikalisch trocknende Polymere bzw. deren Lösungen in organischen Lösungsmitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Die photopolymerisierbaren Gemische können ausser dem Photoinitiator verschiedene Additive enthalten. Beispiele hierfür sind thermische Inhibitoren, die eine vorzeitige Polymerisation verhindern sollen, wie z.B. Hydrochinon oder sterisch gehinderte Phenole. Zur Erhöhung der Dunkellagerstabilität können z.B. Kupferverbindungen, Phosphorverbindungen, quartäre Ammoniumverbindungen oder Hydroxylaminderivate verwendet werden. Zwecks Ausschluss des Luftsauerstoffes während der Polymerisation kann man Paraffin oder ähnliche wachsartige Stoffe zusetzen, die bei Beginn der Polymerisation an die Oberfläche wandern. Als Lichtschutzmittel können in geringer Menge UV-Absorber, wie z.B. solche vom Benztriazol-, Benzophenon- oder Oxalanilid-Typ, zugesetzt werden. Noch besser ist der Zusatz von Lichtschutzmitteln, die UV-Licht nicht absorbieren, wie z.B. von sterisch gehinderten Aminen (HALS).

In bestimmten Fällen kann es von Vorteil sein, Gemische von zwei oder mehr der Photoinitiatoren der Formel IV zu verwenden. Selbstverständlich können auch Gemische mit bekannten Photoinitiatoren verwendet werden, z.B. Gemische mit Benzophenon, Acetophenonderivaten, Benzoinethern, Benzilketalen, Monoacrylphosphinoxiden oder Bisacylphosphinoxiden.

Zur Beschleunigung der Photopolymerisation können Amine zugesetzt werden, wie z.B. Triethanolamin, N-Methyl-diethanolamin, p-Dimethylaminobenzoesäure-ethylester, Michlers Keton oder Bisdiethylaminobenzophenon. Die Wirkung der Amine kann verstärkt werden durch den Zusatz von aromatischen Ketonen vom Typ des Benzophenons.

Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Benzophenon-, Thioxanthon-, Anthrachinon- und 3-Acylcumarinderivate sowie 3-(Aroylmethylen)-thiazoline.

Die Wirksamkeit der Photoinitiatoren lässt sich steigern durch Zusatz von Titanocenderivaten mit fluororganischen Resten, wie sie in den EP-A-122'223, 186'626 und 318894 beschrieben sind, z.B. in einer Menge von 0,1 - 20 %. Beispiele für solche Titanocene sind Bis(methylcyclopentadienyl)-bis-(2,3,6-trifluorphenyl)-titan, Bis(cyclopentadienyl)-bis(4-di-butylamino-2,3,5,6-tetrafluorphenyl)-titan, Bis(methylcyclopentadienyl)-2-(trifluormethyl)-phenyl-titan-isocyanat, Bis(cyclopentadienyl)-2-(trifluormethyl)phenyl-titan-trifluoracetat, Bis(methylcyclopentadienyl)-bis(4-decyloxy-2,3,5,6-tetrafluorphenyl)-titan, Bis(cyclopentadienyl)-bis-[2,6-difluor-3-(pyrr-1-yl)phenyl]-titan, Bis(methylcyclopentadienyl)-bis-[2,6-difluor-3-(pyrr-1-yl)phenyl]-titan, Bis(cyclopentadienyl)-bis-[2,6-difluor-3-(2,5-dimethyl-pyrr-1-yl)phenyl]-titan und Bis(methylcyclopentadienyl)-bis-[2,6-difluor-3-(2,5-dimethyl-pyrr-1-yl)phenyl]-titan. Für diese Gemische eignen sich vor allem flüssige α-Aminoketone.

Die photopolymerisierbare Zusammensetzung, enthaltend
A) mindestens eine ethylenisch ungesättigte photopolymerisierbare Verbindung, und
B) mindestens einen Photoinitiator der Formel IV sowie
C) gegebenenfalls weitere bekannte und übliche Zusatzstoffe
kann für verschiedene Zwecke verwendet werden. In erster Linie ist ihre Verwendung in pigmentierten oder eingefärbten Systemen von Bedeutung, wie z.B. für Druckfarben, für photographische Reproduktionsverfahren, Bildaufzeichnungsverfahren und zur Herstellung von Reliefformen.

Ein weiteres wichtiges Einsatzgebiet sind Anstrichstoffe, die pigmentiert oder unpigmentiert sein können. Besonders wertvoll sind die Gemische in Weisslacken, worunter man durch TiO₂ pigmentierte Anstrichstoffe versteht. Das in den photohärtbaren Massen enthaltene Pigment kann ein anorganisches Pigment sein, wie z.B. Titandioxid (Rutil oder Anatas), Eisenoxidgelb, Eisenoxidrot, Chromgelb, Chromgrün, Nickeltitangelb, Ultramarinblau, Kobaltblau, Cadmiumgelb, Cadmiumrot oder Zinkweiss. Das Pigment kann ein organisches Pigment sein, wie z.B. ein Mono- oder Bisazopigment oder ein Metallkomplex davon, ein Phthalocyaninpigment, ein polycyclisches Pigment, wie z.B. ein Perylen-, Thioindigo-, Flavanthron-, Chinacridon-, Tetrachlorisoindolinon- oder Triphenylmethan-Pigment. Das Pigment kann auch ein Russ sein oder ein Metallpulver, wie z.B. Aluminium- oder Kupferpulver. Das Pigment kann auch ein Gemisch von zwei oder mehreren verschiedenen Pigmenten sein, wie es zur Erzielung bestimmter Farbtöne üblich ist.

Das Pigment kann in einer Menge von 5 bis 60 Gew.%, bezogen auf die gesamte Masse, vorliegen; in Druckfarben liegen meist 10 - 30 % Pigment vor.

Weitere Einsatzgebiete sind die Strahlenhärtung von Photoresists, die Photovemetzung silberfreier Filme sowie die Herstellung von Druckplatten. Eine weitere Verwendung ist die für Aussenanstriche, die im Tageslicht oberflächlich nachhärten. In Photresist oder reprographischen Filmen werden zur Farbgebung statt Pigmenten auch häufig Farbstoffe verwendet. Hierbei kann es sich um organische Farbstoffe der verschiedensten Klassen handeln, beispielsweise Azofarbstoffe, Methinfarbstoffe, Anthrachinon-Farbstoffe oder Metallkomplexfarbstoffe. Diese Farbstoffe sind in den verwendeten Konzentrationen in den jeweiligen Bindemitteln löslich. Die üblichen Konzentrationen sind 0,1 bis 20 %, vorzugsweise 1-5 Gew.%, bezogen auf die gesamte Masse.

Die Photoinitiatoren werden für die angeführten Anwendungsgebiete zweckmässig in Mengen von 0,1 bis 20 Gew.%, vorzugsweise etwa 0,5 bis 5 Gew.%, bezogen auf die photopolymerisierbare Zusammensetzung, angewendet.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, superaktinische Leuchtstoffröhren, Metallhalogenid-Lampen oder Laser geeignet, deren Emissionsmaxima im Bereich zwischen 250 und 450 nm liegen. Im Falle einer Kombination mit Photosensibilisatoren oder Ferrocenderivaten kann auch längerwelliges Licht oder Laserstrahlen bis 600 nm verwendet werden.

Die folgenden Beispiele veranschaulichen die Erfindung ohne sie darauf zu limitieren.

Beispiel 1: In einem 1 I Hochdruckreaktor werden 181,7 g (0,80 Mol) 1-(4-Bromphenyl)-butan-1-on und 348,5 g (4,0 Mol) Morpholin purum und 72,0 g (4,0 Mol) deionisiertes Wasser vorgelegt. Der Reaktor wird verschlossen und die Lösung wird in ca. 90 Minuten auf 170°C geheizt. Der Innendruck steigt von Null auf 5-6 bar und stabilisiert sich eine Stunde später bei 4-5 bar. Die Reaktionslösung wird ca. 28 Stunden bei ca. 170°C weitergerührt. Dann wird die Reaktionslösung abgekühlt und bei ca. 80°C dem Reaktor entnommen.

Die Reaktionslösung wird in einer Destillationsapparatur auf ca. 104°C erwärmt, um das Wasser abzudestillieren. Dann wird unter schwachem Vakuum das Morpholin abdestilliert. Nach beendeter Destillation wird 144,0 g (0,80 Mol) Natriummethylatlösung à 30 % in Methanol zugesetzt und die Suspension wird aufgeheizt, um das Methanol abzudestillieren. Nach beendeter Methanoldestillation wird die Reaktionsmischung evakuiert und das Morpholin abdestilliert. Bei ca. 80°C werden dann 90 g deionisiertes Wasser zugegeben und verrührt. Das Wasser wird dann abgetrennt. Die überstehende Phase (ca. 196 g Rohausbeute, ca. 105 % der Theorie) wird mit 150 ml (117,5 g) Isopropanol verdünnt, abgekühlt und zur Kristallisation angeimpft. Die Suspension wird bei ca. -10°C filtriert und mit kaltem Isopropanol nachgewaschen. Es werden 148,7 g 1-(4-Morpholinophenyl)-butan-1-on (79,6 % der Theorie) als hellbeige Kristalle mit einem Smp. 64,5°C - 65,5°C erhalten. Reinheit: >99,0 %.

| Elementaranalyse: | | | | | |
|---|---|---|---|---|---|
| | %C | | %H | | % N |
| berechnet: | 72,07 | berechnet: | 8,21 | berechnet: | 6,00 |
| gefunden: | 72,03 | gefunden: | 8,29 | gefunden: | 5,92 |

Aus dem Filtrat (Isopropanol) lassen sich weitere 19,8 g (ca. 10 % der Theorie) an 1-(4-Morpholinophenyl)-butan-1-on gewinnen.

Arbeitet man analog nach der Arbeitsweise von Beispiel 1, verwendet aber äquimolekulare Mengen der cyclischen Amine gemäss Tabelle 1 und äquimolekulare Mengen an 1-(4-Bromphenyl)-alkyl-keton gemäss Tabelle 1 so werden die cyclischen Amin substituierten Phenyl-alkyl-Keton-Verbindungen gemäss der Tabelle 1 erhalten, deren physikalische Analysendaten ebenfalls in der Tabelle 1 angegeben sind.

**Tabelle 1**

| Bsp. | Cyclisches Amin | 1-(4-Bromphenyl)-alkyl-Keton | Cyclisches Amin substituiertes Phenylalkyl-Keton | Physikalische Analysendaten | | | |
|---|---|---|---|---|---|---|---|
| | | | | Smp. °C | %C | %H | %N |
| 2 | 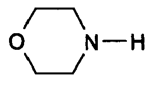 | 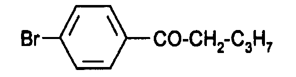 | 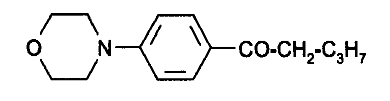 | 68,5-70,5 | ber. 72,84 gef. 72,86 | 8,56 8,76 | 5,66 5,36 |
| 3 | 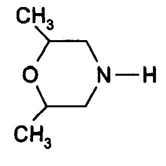 | 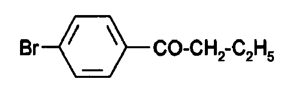 | 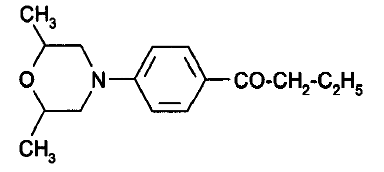 | 90,2-91,7 | ber. 73,54 gef. 73,57 | 8,87 8,85 | 5,36 5,28 |
| 4 | 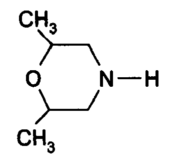 | 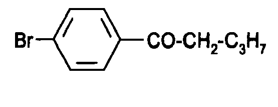 | 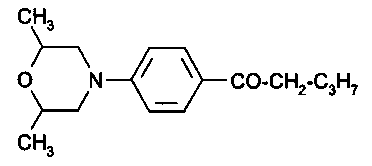 | 76,7-77,3 | ber. 74,15 gef. 74,15 | 9,15 9,18 | 5,09 5,04 |
| 5 | 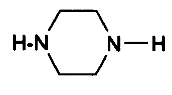 | 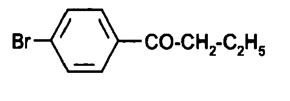 | 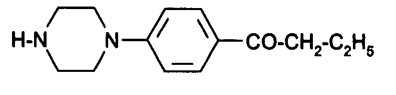 | 67-69 | ber.72,38 gef. 72,44 | 8,68 8,51 | 12,06 12,01 |
| 6 | 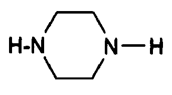 | 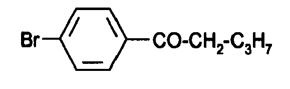 | 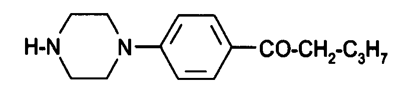 | 61-65,2 | ber. 73,13 gef. 73,22 | 9,00 9,06 | 11,37 11,35 |
| 7 | 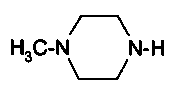 | 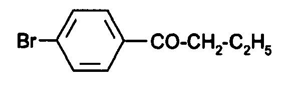 | 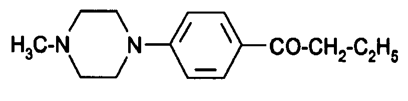 | 96-98 | ber. 73,13 gef. 73,17 | 9,00 9,03 | 11,37 11,34 |
| 8 | 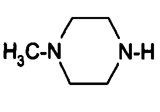 | 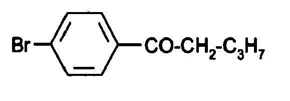 | 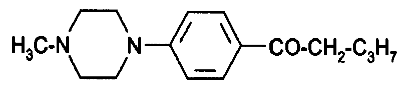 | 74,1-76,8 | ber. 73,81 gef. 74,04 | 9,29 9,27 | 10,76 10,65 |
| 9 | 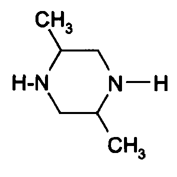 | 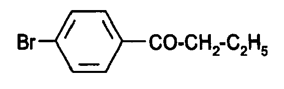 | 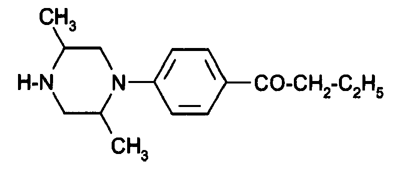 | 74,6-76,7 | ber. 73,81 gef. 74,03 | 9,29 9,55 | 10,76 10,76 |
| 10 | 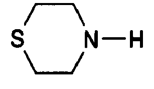 | 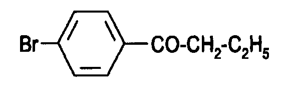 | 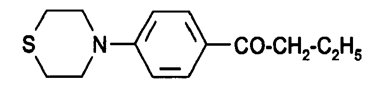 | 27-31 | ber. 67,43 gef. 67,50 | 7,68 7,85 | ** 5,62 5,75 |
| 11 | 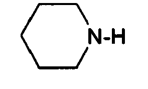 | 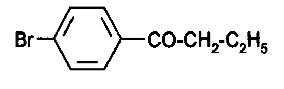 | 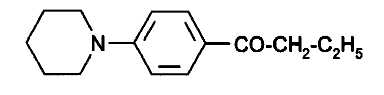 | 47,5-49,5 | ber. 77,88 gef. 77,66 | 9,15 9,27 | 6,05 5,95 |
| 12 | 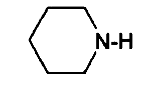 | 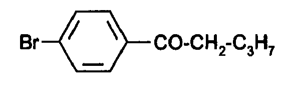 | 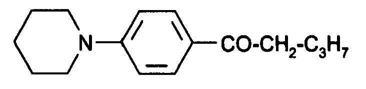 | 56,8-60,1 | ber. 78,32 gef. 78,33 | 9,45 9,42 | 5,71 5,57 |
| 13 | 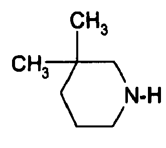 | 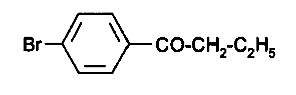 | 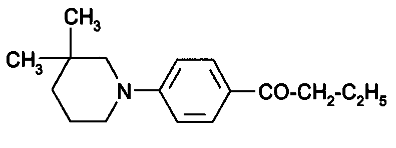 | 47-48,7 | ber. 78,72 gef. 78,61 | 9,71 9,90 | 5,40 5,31 |
| 14 | 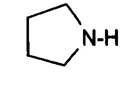 | 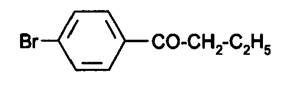 | 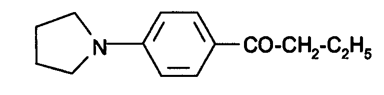 | 83,5-85,3 | ber. 77,38 gef. 77,25 | 8,81 8,89 | 6,45 6,40 |
| 15 | 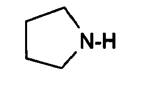 | 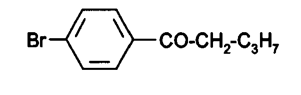 | 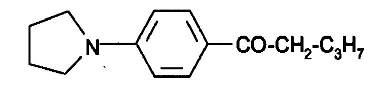 | 83-84,7 | ber. 77,88 gef. 77,80 | 9,15 9,34 | 6,05 6,01 |
| 16 | 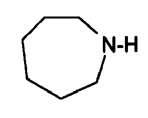 | 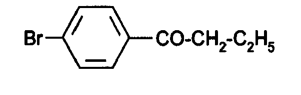 | 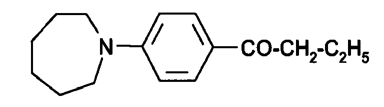 | 32-34 | ber. 78,32 gef. 78,23 | 9,45 9,37 | 5,71 5,64 |
| 17 | 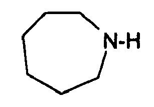 | 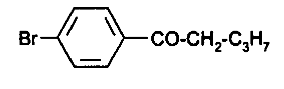 | 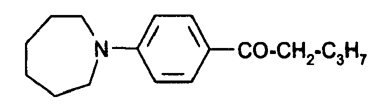 | 55,5-57,2 | ber. 78,72 gef. 78,61 | 9,71 9,83 | 5,40 5,53 |
| 18 | 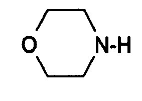 | 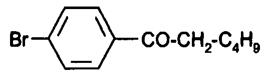 | 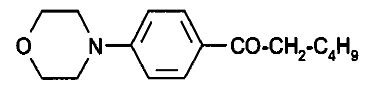 | 66-67 | ber. 73,53 gef. 73,53 | 8,87 8,85 | 5,36 5,25 |
| 19 | 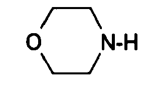 | 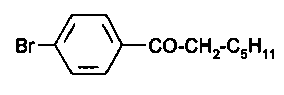 | 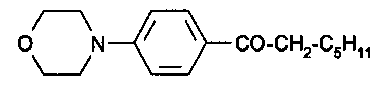 | 55-56 | ber. 74,14 gef. 74,17 | 9,15 9,22 | 5,09 4,93 |
| 20 | 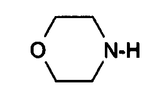 | 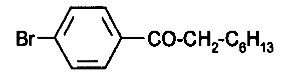 | 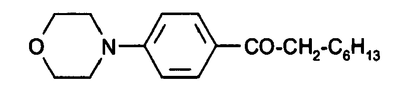 | 59-60 | ber. 74,70 gef. 74,20 | 9,40 9,38 | 4,84 4,41 |
| 21 | 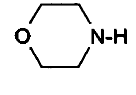 | 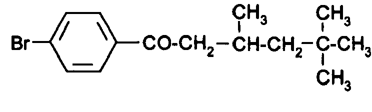 | 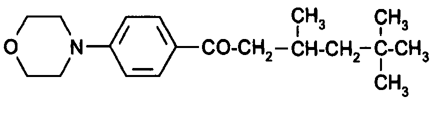 | 76-77,8 | ber. 75,21 gef. 75,25 | 9,63 9,69 | 4,61 4,31 |

| | | | | | | | |
|---|---|---|---|---|---|---|---|
| ** % S berechnet: 12,86 gefunden: 12,97 | | | | | | | |

Beispiel 22: In einem 1 I Hochdruckreaktor werden 392,1 g (4,50 Mol) Morpholin purum und 162,0 g (9,00 Mol) deionisiertes Wasser vorgelegt. Der Reaktor wird verschlossen und die Lösung wird unter Rühren in ca. 1 Stunde auf 220°C geheizt, wobei der Innendruck auf 20 bar steigt. Dann werden während fünf Stunden 164,4 g (0,90 Mol) 1-(4-Chlorphenyl)-butan-1-on gleichmässig bei 220°C zudosiert. Am Ende des Zudosierens ist der Druck auf ca. 18 bar gefallen und die Reaktion ist über 80 % abgelaufen. Das Reaktionsgemisch wird weitere fünf Stunden bei 220°C nachgerührt. Der Druck sinkt langsam auf 17 bar. Dann lässt man auf 80°C abkühlen.

Mit 75,6 g (0,945 Mol) Natronlauge ä 50 % wird das Morpholinsalz neutralisiert. Unter reduziertem Vakuum wird bei 80°C - 100°C ein Morpholin-Wasser-Gemisch abdestilliert. Dann werden 180 g deionisiertes Wasser und 203 g Siedegrenzenbenzin (110°C - 140°C Siedebereich) zugegeben. Das Gemisch wird bei 80°C über wenig Aktivkohle klarfiltriert. Die Wasserphase wird bei 80°C abgetrennt. Das Produkt wird aus dem Siedegrenzenbenzin auskristallisiert, filtriert und getrocknet. Die Ausbeute an Endprodukt beträgt 200,6 g 1-(4-Morpholinophenyl)-butan-1-on (ca. 95,5 % der Theorie). Das beige Produkt hat eine Reinheit von >99,0 %, der Schmelzpunkt liegt bei 64,8°C. Im Filtrat sind nur Produkt und Edukt feststellbar.

| Elementaranalyse: | | | | | |
|---|---|---|---|---|---|
| | %C | | %H | | % N |
| berechnet: | 72,07 | berechnet: | 8,21 | berechnet: | 6,00 |
| gefunden: | 72,09 | gefunden: | 8,26 | gefunden: | 5,86 |

Beispiel 23: In einem 1 I Hochdruckreaktor werden 392,1 g (4,50 Mol) Morpholin purum und 162,0 g (9,00 Mol) deionisiertes Wasser vorgelegt. Der Reaktor wird verschlossen und in ca. 60 Minuten auf 215°C - 220°C aufgeheizt. Der Druck erreicht 19,9 bar. Dann werden 164,4 g (0,90 Mol) 1-(4-Chlorphenyl)-butan-1-on in flüssiger Form mit einer Druckpumpe zudosiert und die Temperatur bei 215°C - 220°C gehalten.

Dauer der Dosierzeit: 3 Stunden. Der Druck sinkt auf 18,5 bar. Anschliessend wird während 3 weiteren Stunden bei 215°C - 220°C nachgerührt. Der Druck sinkt auf 17,8 bar. Anschliessend wird auf ca. 80°C abgekühlt.

Die Reaktionslösung wird in eine Destillationsapparatur transferiert und mit 36,0 g (0,90 Mol) Natriumhydroxid in Perlenform versetzt. Dann wird das Wasser zwischen 70°C und 90°C unter reduziertem Druck abdestilliert und anschliessend auch das Morpholin. Das Endvacuum ist ca. 30 mbar. Anschliessend wird die Apparatur mit Stickstoff entlastet und es werden bei ca. 88°C 171,8 g deionisiertes Wasser und 30,2 g Toluol zugesetzt. Nach dem Verrühren wird das Wasser abgetrennt und das Toluol abdestilliert. Die Reaktionslösung wird warm mit 152,9 g Isopropanol versetzt und bei ca. 65°C heiss über einen Druckfilter geklärt. Die Isopropanollösung wird gekühlt und angeimpft. Die Suspension wird bei ca. 0°C filtriert und mit kaltem Isopropanol nachgewaschen. Es werden 186,7 g 1-(4-Morpholinophenyl)-butan-1-on (88,9 % der Theorie) als hellbeige Kristalle mit einem Smp.: 64,4°C - 65,5°C erhalten.

Beispiel 24: In einem 1 I Hochdruckreaktor werden 164,4 g (0,90 Mol) 1-(4-Chlorphenyl)-butan-1-on, 392,1 g (4,50 Mol) Morpholin purum, 162,0 g (9,00 Mol) deionisiertes Wasser und 0,89 g (0,90 mMol) Kupfer-I-chlorid vorgelegt. Der Reaktor wird verschlossen und die Lösung wird unter Rühren in ca. 1 Stunde auf 180°C geheizt. Dann wird langsam weitergeheizt und pro Stunde die Temperatur um ca. 10°C erhöht. In vier Stunden werden 220°C und ein Druck von 20 bar erreicht. Dann lässt man weitere fünf Stunden bei 220°C nachreagieren. Der Druck sinkt dabei langsam auf 17 bar. Dann lässt man auf 80°C abkühlen.

Mit 75,6 g (0,945 Mol) Natronlauge à 50 % wird das Morpholinsalz neutralisiert und der Katalysator gefällt. Unter reduziertem Vakuum wird bei 80°C - 100°C ein Morpholin-Wasser-Gemisch abdestilliert. Dann werden 180 g deionisiertes Wasser und 203 g Siedegrenzenbenzin (110°C - 140°C Siedebereich) zugegeben. Das Gemisch wird bei 80°C über wenig Aktivkohle klarfiltriert um den Katalysator zu entfernen. Die Wasserphase wird bei 80°C abgetrennt. Das Produkt wird aus dem Siedegrenzenbenzin auskristallisiert, filtriert und getrocknet. Die Ausbeute an Endprodukt beträgt 199,8 g 1-(4-Morpholinophenyl)-butan-1-on (ca. 95,2 % der Theorie). Das beige Produkt besitzt eine Reinheit von >99,0 %, der Schmelzpunkt liegt bei 64,8°C. Im Filtrat sind nur Produkt und Edukt feststellbar.

### Beispiel 25:

### a) 2-Brom-1-(4-morpholinophenyl)-butan-1-on

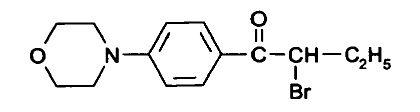

In einem 2,5 I Sulfierkolben werden 466,6 g (2 Mol) 1-(4-Morpholinophenyl)-butan-1-on gemäss Beispiel 1 in 600 ml (10,5 Mol) Eisessig gelöst. Die Temperatur sinkt dabei auf 5°C. Unter schwacher Kühlung werden in ca. 2,5 Stunden bei Raumtemperatur 319,6 g (2 Mol) Brom zugetropft. Das Ende der Bromierung wird mit einem Dünnschichtchromatogramm überprüft. Dann wird die Reaktionslösung mit 300 g Eis versetzt und dazu wird unter guter Kühlung in einer Stunde eine Natronlaugenlösung, hergestellt aus 1600 g (12 Mol) Natronlauge und 600 g Eis, zugetropft. Die gelbe Suspension hat ca. pH 6, wird filtriert und mit Wasser nachgewaschen. Die Kristalle werden getrocknet. Sie schmelzen bei 99°C bis 102°C. Die Ausbeute ist 631,2 g von 2-Brom-1-(4-morpholinophenyl)-butan-1-on. Das ¹H-NMR-Spektrum des Rohproduktes stimmt mit der angegebenen Struktur überein.

| Elementaranalyse: | %C | %H | %N | %Br |
|---|---|---|---|---|
| berechnet: | 53,86 | 5,81 | 4,49 | 25,59 |
| gefunden: | 53,23 | 5,73 | 4,24 | 25,50 |

### b) 2-Dimethylamino-1-(4-morpholinophenyl)-butan-1-on

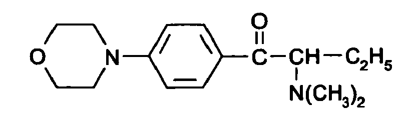

In einem 2,5 l Sulfierkolben werden 312,2 g (1 Mol) 2-Brom-1-(4-morpholinophenyl)-butan-1-on gemäss a) oben mit 600 ml Methylethylketon versetzt und unter Rühren auf 50°C erwärmt. Zur erhaltenen Lösung gibt man 207,3 g (1,5 Mol) Kaliumcarbonat und leitet bei 50°C innerhalb 1,5 Stunden unter Niveau 56,6 g (1,3 Mol) gasförmiges Dimethylamin in die Suspension ein. Dann lässt man noch weitere 4 bis 5 Stunden nachreagieren, bis im Dünnschichtchromatogramm kein Edukt mehr nachweisbar ist. Die Suspension wird dann mit 550 ml Wasser versetzt und verrührt. Die wässrige Phase wird abgetrennt und die 900 ml organische Phase, enthaltend 2-Dimethylamino-1-(4-morpholinophenyl)-butan-1-on, wird unverändert in der nächsten Reaktionsstufe eingesetzt.
In einem Parallelversuch wird die organische Phase eingeengt. Die erhaltenen Kristalle werden aus Hexan umkristallisiert. Nach dem Trocknen erhält man 235,1 g hellgelbe Kristalle, die bei 53°C bis 56°C schmelzen. Das ¹H-NMR-Spektrum des Produktes, 2-Dimethylamino-1-(4-morpholinophenyl)-butan-1-on, stimmt mit der angegebenen Struktur überein.

| Elementaranalyse: | %C | %H | %N |
|---|---|---|---|
| berechnet: | 69,53 | 8,75 | 10,14 |
| gefunden: | 68,91 | 8,59 | 9,74 |

### c) 2-Benzyl-2-dimethylamino-1-(4-morpholinophenyl)-butan-1-on

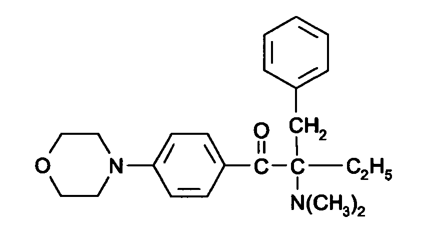

In einem 2,5 I Sulfierkolben werden 900 ml Lösung (1 Mol) von 2-Dimethylamino-1-(4-morpholinophenyl)-butan-1-on gemäss b) oben wieder auf 50°C erwärmt. Dann werden in 20 Minuten 179,7 g (1,05 Mol) Benzylbromid zugetropft. Dann lässt man noch weitere 3 bis 4 Stunden bei 50°C nachrühren, bis im Dünnschichtchromatogramm kein Edukt mehr nachweisbar ist. Nun wird die Temperatur auf 60°C erhöht und innert 45 Minuten werden portionenweise 80 g (2 Mol) Natriumhydroxidpulver zugegeben. Anschliessend wird noch 1 bis 2 Stunden bei 50°C weitergerührt, bis im Dünnschichtchromatogramm kein Edukt mehr nachweisbar ist. Das Reaktionsgemisch wird mit 150 ml Wasser versetzt und verrührt. Die Wasserphase wird abgetrennt und die organische Phase am Vacuumrotationsverdampfer eingeengt. Im Kolben bleiben 378,3 g gelbliches Rohprodukt von 2-Benzyl-2-dimethylamino-1-(4-morpholinophenyl)-butan-1-on das bei 102°C bis 110°C schmilzt. Das Rohprodukt wird in 600 ml Ethanol heiss gelöst, abgekühlt, kristallisiert, filtriert und mit kaltem Ethanol nachgewaschen. Die Kristalle werden getrocknet. Sie schmelzen bei 114°C bis 115°C und sind im Gas- und im Dünnschicht-chromatogramm rein. Die Ausbeute ist 299,0 g von 2-Benzyl-2-dimethylamino-1-(4-morpholinophenyl)-butan-1-on. Aus der Mutterlauge lassen sich weitere 22,4 g an reinem Produkt isolieren. Das ¹H-NMR-Spektrum des Reinproduktes stimmt mit der angegebenen Struktur überein.

| Elementaranalyse: | %C | %H | %N |
|---|---|---|---|
| berechnet: | 75,38 | 8,25 | 7,64 |
| gefunden: | 75,23 | 8,21 | 7,58 |

## Patentansprüche

1. Verfahren zur Herstellung von Verbindungen der Formel I 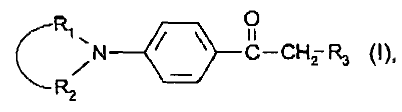 worin bedeuten:
**R₁** und **R₂** zusammen unverzweigtes oder verzweigtes, unsubstituiertes oder substituiertes C₃-C₂₀-Alkylen, welches durch ein oder mehrere -O-, -S- oder -N(R₄)-Gruppen unterbrochen sein kann,
**R₃** unverzweigtes oder verzweigtes unsubstituiertes **C₂-C₁₀-Alkyl,** und
**R₄** Wasserstoff, unverzweigtes oder verzweigtes C₁-C₃-Alkyl, unverzweigtes oder verzweigtes C₃-C₅-Alkenyl, C₇-C₉-Phenylalkyl, C₁-C₄-Hydroxyalkyl oder Phenyl, durch Aminolyse eines p-Halogenphenylalkylketons der Formel II 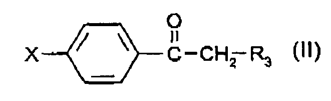 mit einem cyclischen Amin der Formel III 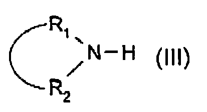 in Wasser bei einer Temperatur, von mindestens 130°C, und, **dadurch gekennzeichnet, dass** die Reaktion unter Druck in einem Druckkessel durchgeführt wird,
in welchen Formeln **X** ein Halogenatom bedeutet und R₁, R₂ und R₃ die unter Formel I angegebene Bedeutung haben.

2. Verfahren gemäss Anspruch 1, **gekennzeichnet durch** die Verwendung eines p-Halogenphenylalkylketons der Formel II, worin X Chlor oder Brom bedeutet und R₃ die unter Formel I angegebene Bedeutung hat.

3. Verfahren gemäss der Ansprüche 1 und 2, **dadurch gekennzeichnet, dass** die Menge an cyclischem Amin der Formel III zwischen etwa 2,5-12 Moläquivalente bezogen auf das p-Halogenphenylalkylketon der Formel II beträgt.

4. Verfahren gemäss der Ansprüche 1 bis 3, **dadurch gekennzeichnet, dass** die Menge an Wasser zwischen etwa 1-100 Moläquivalenten bezogen auf das p-Halogenphenylalkylketon der Formel II beträgt.

5. Verfahren gemäss den Ansprüchen 1 bis 4, **dadurch gekennzeichnet, dass** die Temperatur in einem Bereich von etwa 140°C - 240°C liegt.

6. Verfahren gemäss den Ansprüchen 1 bis 5, **dadurch gekennzeichnet, dass** das p-Halogenphenylalkylketon der Formel II im Reaktionsgefäss zusammen mit dem Wasser und dem cyclischen Amin vorgelegt wird und entweder sofort oder langsam während der Reaktion bis zur Endtemperatur aufgeheizt wird.

7. Verfahren gemäss den Ansprüchen 1 bis 6, **dadurch gekennzeichnet, dass** das p-Halogenphenylalkylketon der Formel II während der Reaktion zum Wasser und dem cyclischen Amin, das vorgängig auf die Reaktionstemperatur geheizt wurde, zudosiert wird.

8. Verfahren gemäss Anspruch 1, **dadurch gekennzeichnet, dass** 1 Teil p-Bromphenylalkylketon oder 1 Teil p-Chlorphenylalkylketon der Formel II, worin **R₃** unverzweigtes oder verzweigtes, unsubstituiertes C₂-C₇-Alkyl bedeutet mit 5 Teilen eines cyclischen Amins der Formel III, worin **R₁** und **R₂** zusammen C₄-C₆-Alkylen bedeuten, das durch eine -O-, -S- oder N(R₄)-Gruppe unterbrochen sein kann und **R₄** Wasserstoff oder Methyl bedeutet und 5 Teile Wasser im Reaktionsgefäss vorgelegt werden und unter Druck bei einer Temperatur von etwa 160°C - 180°C im Fall von p-Bromphenylalkylketon bzw. von etwa 200°C - 230°C im Fall von p-Chlorphenylalkylketon umgesetzt werden.

9. Verfahren gemäss Anspruch 1, **dadurch gekennzeichnet, dass** 10 bis 20 Teile einescyclischen Amins der Formel III, worin **R₁** und **R₂** zusammen C₄-C₆-Alkylen bedeuten, das durch eine -O-, -S- oder -N(R₄)-Gruppe unterbrochen sein kann und **R₄** Wasserstoff oder Methyl bedeutet und 20 bis 40 Teile Wasser im Reaktionsgefäss vorgelegt werden, 2 bis 4 Teile p-Chlorphenylalkylketon der Formel II, worin **R₃** unverzweigtes oder verzweigtes, unsubstituiertes C₂-C₇-Alkyl bedeutet, zudosiert werden und unter Druck bei etwa 210°C - 230°C umgesetzt werden.

## Claims

1. A process for preparing compounds of the formula I 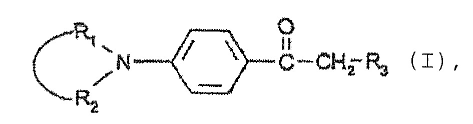 in which:
R₁ and R₂ together are unbranched or branched, unsubstituted or substituted C₃-C₂₀ alkylene which may be interrupted by one or more -O-, -S- or -N(R₄)-groups,
R₃ is unbranched or branched, unsubstituted C₂-C₁₀ alkyl, and
R₄ is hydrogen, unbranched or branched C₁-C₃ alkyl, unbranched or branched C₃-C₅ alkenyl, C₇-C₉ phenylalkyl, C₁-C₄ hydroxyalkyl or phenyl, by aminolysis of a p-halophenyl alkyl ketone of the formula II 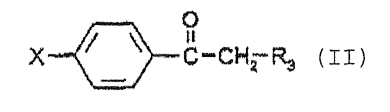 with a cyclic amine of the formula III 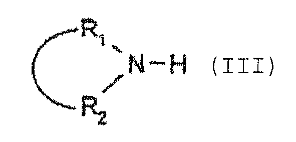 in water at a temperature of at least 130°C, which comprises carrying out the reaction under pressure in a pressure vessel,
in which formulae X is a halogen atom and R₁, R₂ and R₃ are as defined under formula I.

2. The process according to claim 1, using a p-halophenyl alkyl ketone of the formula II in which X is chlorine or bromine and R₃ is as defined under formula I.

3. The process according to claims 1 and 2, wherein the amount of cyclic amine of the formula III is between about 2.5-12 molar equivalents, based on the p-halophenyl alkyl ketone of the formula II.

4. The process according to claims 1 to 3, wherein the amount of water is between about 1-100 molar equivalents, based on the p-halophenyl alkyl ketone of the formula II.

5. The process according to claims 1 to 4, wherein the temperature is in a range of about 140°C - 240°C.

6. The process according to claims 1 to 5, wherein the p-halophenyl alkyl ketone of the formula II is charged to the reaction vessel together with the water and the cyclic amine, and heating takes place, either immediately or slowly during the reaction, until the final temperature is reached.

7. The process according to claims 1 to 6, wherein the p-halophenyl alkyl ketone of the formula II is metered in during the reaction to the water and the cyclic amine, which has been heated to the reaction temperature beforehand.

8. The process according to claim 1, wherein one part of p-bromophenyl alkyl ketone or one part of p-chlorophenyl alkyl ketone of the formula II in which R₃ is unbranched or branched, unsubstituted C₂-C₇ alkyl is charged with 5 parts of a cyclic amine of the formula III in which R₁ and R₂ together are C₄-C₆ alkylene which may be interrupted by an -O-, -S- or -N(R₄)- group and R₄ is hydrogen or methyl, and 5 parts of water to the reaction vessel and this initial charge is reacted under pressure at a temperature of about 160°C - 180°C in the case of p-bromophenyl alkyl ketone or of about 200°C - 230°C in the case of p-chlorophenyl alkyl ketone.

9. The process according to claim 1, wherein 10 to 20 parts of a cyclic amine of the formula III in which R₁ and R₂ together are C₄-C₆ alkylene which may be interrupted by an -O-, -S- or -N(R₄)- group and R₄ is hydrogen or methyl, and 20 to 40 parts of water, are charged to the reaction vessel, 2 to 4 parts of p-chlorophenyl alkyl ketone of the formula II in which R₃ is unbranched or branched, unsubstituted C₂-C₇ alkyl are metered in, and the reactants are reacted under pressure at about 210°C - 230°C.

## Revendications

1. Procédé pour la préparation de composés de formule I, 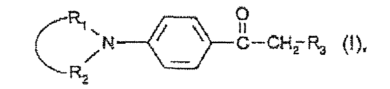 dans laquelle :
R₁ et R₂ signifient, ensemble, C₃-C₂₀-alkylène non ramifié ou ramifié, non substitué ou substitué, qui peut être interrompu par un ou plusieurs groupes -O-, -S- ou -N(R₄)-,
R₃ signifie C₂-C₁₀-alkyle non ramifié ou ramifié, non substitué, et
R₄ signifie hydrogène, C₁-C₃-alkyle non ramifié ou ramifié, C₃-C₅-alcényle non ramifié ou ramifié, C₇- C₉-phénylalkyle, C₁-C₄-hydroxyalkyle ou phényle, par aminolyse d'une p-halogénophénylalkylcétone de formule II 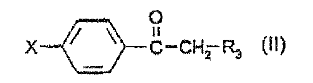
avec une amine cyclique de formule III 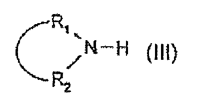 dans l'eau à une température d'au moins 130°C, et **caractérisé en ce que** la réaction est réalisée sous pression dans une cuve sous pression,
et dans ces formules, X signifie un atome d'halogène et R₁, R₂ et R₃ ont la signification indiquée pour la formule I.

2. Procédé selon la revendication 1, **caractérisé par** l'utilisation d'une p-halogénophénylalkylcétone de formule II, où X signifie chlore ou brome et R₃ a la signification indiquée pour la formule I.

3. Procédé selon les revendications 1 et 2, **caractérisé en ce que** la quantité d'amine cyclique de formule III est située entre environ 2,5 et 12 équivalents en mole par rapport à la p-halogénophénylalkylcétone de formule II.

4. Procédé selon les revendications 1 à 3, **caractérisé en ce que** la quantité d'eau est située entre environ 1 et 100 équivalents en mole par rapport à la p-halogénophénylalkylcétone de formule II.

5. Procédé selon les revendications 1 à 4, **caractérisé en ce que** la température se situe dans une plage d'environ 140°C-240°C.

6. Procédé selon les revendications 1 à 5, **caractérisé en ce que** la p-halogénophénylalkylcétone de formule II est disposée au préalable dans le récipient de réaction, ensemble avec l'eau et l'amine cyclique et est chauffée soit directement, soit lentement, pendant la réaction jusqu'à la température finale.

7. Procédé selon les revendications 1 à 6, **caractérisé en ce que** la p-halogénophénylalkylcétone de formule II est ajoutée en dosant pendant la réaction à l'eau et à l'amine cyclique qui a été chauffée au préalable à la température de réaction.

8. Procédé selon la revendication 1, **caractérisé en ce qu'**on dispose au préalable dans le récipient de réaction 1 partie de p-bromophénylalkylcétone ou 1 partie de p-chlorophénylalkylcétone de formule II, où R₃ signifie C₂-C₇-alkyle non ramifié ou ramifié, non substitué, avec 5 parties d'une amine cyclique de formule III, dans laquelle R₁ et R₂ signifient ensemble C₄-C₆-alkylène, qui peut être interrompu par un groupe -O-, -S- ou -N(R₄)- et R₄ signifie hydrogène ou méthyle et 5 parties d'eau et on les transforme sous pression à une température d'environ 160°C-180°C dans le cas de la p-bromophénylalkylcétone ou d'environ 200°C-230°C dans le cas de la p-chlorophénylalkylcétone.

9. Procédé selon la revendication 1, **caractérisé en ce qu'**on dispose au préalable dans le récipient de réaction 10 à 20 parties d'une amine cyclique de formule III, dans laquelle R₁ et R₂ signifient, ensemble, C₄-C₅-alkylène, qui peut être interrompu par un groupe -O-, -S- ou -N(R₄)- et R₄ signifie hydrogène ou méthyle et 20 à 40 parties d'eau, on ajoute en dosant 2 à 4 parties de p-chlorophénylalkylcétone de formule II, où R₃ signifie C₂-C₇-alkyle non ramifié ou ramifié, non substitué et on transforme sous pression à environ 210°C-230°C.
